# EUROPÄISCHE PATENTSCHRIFT

(11) **EP 0 802 217 B1**
(45) Veröffentlichungstag und Bekanntmachung des Hinweises auf die Patenterteilung: **02.02.2005**
(21) Anmeldenummer: 97106115.5
(22) Anmeldetag: 15.04.1997
(51) Int. Cl.: C08J 5/06, C08K 9/04, C08K 9/06, B60C 1/00, C07F 7/12, C07F 7/18, C07C 321/10, C07C 59/00, C07C 57/00, C07C 233/00, B60C 9/00

(54) **Haftvermittlersubstanz zwischen vulkanisierbarem Polymer und metallischem Festigkeitsträger**
Bonding agent for vulcanizable polymers and metallic reinforcements
Agent adhésif pour polymères vulcanisables et renforcements metalliques

(30) Priorität: 17.04.1996 DE 19615134
(43) Veröffentlichungstag der Anmeldung: 22.10.1997
(73) Patentinhaber: Continental Aktiengesellschaft, 30165 Hannover (DE)
(72) Erfinder: Garnier, Francis, Dr., 94500 Champigny (FR); Lang, Philippe, Dr., 94300 Vincennes (FR); Michalitsch, Richard, Dr., 2305 Kopfstetten 72 (AT); Kaiser, Bernd, Dr., 30419 Hannover (DE); Nauer, Gerhard, Dr., 1210 Wien (AT)

(56) Entgegenhaltungen:
- EP-A- 0 150 168
- EP-A- 0 386 905
- WO-A-90/08170
- DE-B- 2 221 626
- GB-A- 1 177 766
- US-A- 5 126 385
- US-A- 5 466 848
- PATENT ABSTRACTS OF JAPAN vol. 18, no. 528 (C-1258), 6.Oktober 1994 & JP 06 184237 A (HOYA CORP), 5.Juli 1994,
- PATENT ABSTRACTS OF JAPAN vol. 15, no. 48 (C-802), 5.Februar 1991 & JP 02 281001 A (UBE IND LTD), 16.November 1990,
- PATENT ABSTRACTS OF JAPAN vol. 10, no. 214 (C-362), 25.Juli 1986 & JP 61 053261 A (JAPAN TOBACO INC), 17.März 1986,
- DATABASE WPI Section Ch, Week 9614 Derwent Publications Ltd., London, GB; Class A18, AN 96136353 XP002071085 & JP 08 027 213 A (TOSHIBA SILICON KK) , 30.Januar 1996
- DATABASE WPI Section Ch, Week 9431 Derwent Publications Ltd., London, GB; Class A13, AN 94252856 XP002071086 & JP 06 184 237 A (HOYA CORP) , 5.Juli 1994
- DATABASE WPI Section Ch, Week 9346 Derwent Publications Ltd., London, GB; Class A14, AN 93365479 XP002071087 & JP 05 271 642 A (SUNSTAR GIKEN KK) , 19.Oktober 1993
- DATABASE WPI Section Ch, Week 9106 Derwent Publications Ltd., London, GB; Class A85, AN 91039593 XP002071088 & JP 02 305 825 A (ASAHI SCHWEBEL KK) , 19.Dezember 1990
- DATABASE WPI Section Ch, Week 9444 Derwent Publications Ltd., London, GB; Class A14, AN 94-354929 XP002070509 & JP 06 279 732 A (ASAHI GLASS CO LTD) , 4.Oktober 1994

## Beschreibung

Die Erfindung betrifft eine Haftvermittlersubstanz zur besseren Haftung zwischen einem vulkanisierbaren Polymer und einem metallischen Festigkeitsträger, die Verwendung einer Haftvermittlersubstanz zur besseren Haftung zwischen einem vulkanisierbaren Polymer, metallische Festigkeitsträger zur Haftung an vulkanisierbarem Polymer und einen Verbund aus vulkanisierbarem Polymer, einer Haftvermittlersubstanz und einem metallischen Festigkeitsträger.

Um eine ausreichende und lang anhaftende Haftung von Gummi an Metall herzustellen, müssen viele Parameter berücksichtigt werden. So muß die Oberfläche des metallischen Festigkeitsträgers so modifiziert sein, daß eine Anhaftung des Gummis ermöglicht wird. Dazu wurden bis dato metallische Festigkeitsträger (im allgemeinen Stahlcord), die in mit Schwefel vulkanisierbaren Kautschukmischungen eingebettet werden sollten, mit einer Messingschicht überzogen. Weitere Entwicklungen gingen dahin, das Messing durch Zink-Cobalt- bzw. Zink-Nickel-Legierungen zu ersetzen (EP 536 545). Die Messingschicht bzw. Zinklegierung dient dazu, während der Vulkanisation durch Bildung einer Zwischenschicht eine mechanische Verzahnung mit der Gummimischung herzustellen. Ein Nachteil liegt allerdings darin, daß die Zwischenschicht den metallischen Festigkeitsträger (Stahlcord) nicht an allen Stellen bedeckt, so daß diese Stellen Schwachpunkte der Haftung darstellen. Außerdem wird für die Zwischenschichtbildung zusätzlich Schwefel benötigt, der der Kautschukmischung zusätzlich auch zugesetzt werden muß. Um eine beständige Haftung zwischen Gummi und Metall herzustellen, ist es weiterhin erforderlich, daß die Gummimischung sehr alterungsbeständig ist. Dafür wurden Mischungen vorgeschlagen, die Cobaltsalze enthalten. Diese Gummimischungen hatten wiederum den Nachteil, eine geringe Haltbarkeit aufzuweisen. Des weiteren ist für Stahlcordgummierungen bekannt, daß der Zusatz von Resorcin-Formaldehyd-Kondensationsprodukten die Alterungsbeständigkeit der Gummimischung erhöhen soll. Diese Kondensationsprodukte sind aber toxikologisch bedenklich, was deren Verarbeitung erschwert.
Es sind also bereits die vielfältigen Versuche unternommen worden, um die Haftung zwischen Gummi und Metall zu optimieren. So ist z. B. aus der JP 06/279 732 A eine Primerzusammensetzung für die Verbesserung der Haftung zwischen peroxidisch vernetztem Fluorkautschuk und Metall bekannt, die Silankupplungsagenzien enthält. Allerdings ist es jedoch in der Regel immer noch erforderlich den Festigkeitsträger und die Gummimischung der Haftmischung in engen Grenzen aufeinander abzustimmen. Das hat wiederum den Nachteil, daß bei der Kombination einer weiteren angrenzenden Gummischicht die Zusammensetzung dieser an die der Haftmischung angepaßt werden muß und somit nur in engen Grenzen variiert werden kann. Eine Variation der Gummizusammensetzung wirkt sich aber auf die Eigenschaften des Gummiproduktes aus, so daß diese auch nicht optimal eingestellt werden können. Die Notwendigkeit, eine dauerhafte und stabile Haftung zum Stahlcord zu erzielen, schränkt daher die Freiheit ein, andere wünschenswerte Mischungseigenschaften zu optimieren.

Der Erfindung liegt die Aufgabe zugrunde, eine verbesserte Haftung zwischen Gummi und metallischen Festigkeitsträgern herzustellen, die verfahrenstechnisch günstig erzielt werden kann.

Gelöst wird diese Aufgabe durch eine Haftvermittlersubstanz gemäß Anspruch 1, durch die Verwendung einer Haftvermittlersubstanz gemäß Anspruch 5, durch metallische Festigkeitsträger gemäß Anspruch 9 und einen Verbund gemäß Anspruch 12.

Die Erfindung beruht auf der Erkenntnis, daß die oben genannte Verbindungsklasse durch die Funktionalität X eine große Adsorptionskraft zu metallischen Oberflächen aufweist und gleichzeitig durch die Funktionalität Y während der Vulkanisation des Kautschuks eine kovalente Bindung zu diesem hergestellt wird. Durch diese bifunktionelle Verbindungsklasse als Haftvermittler wird eine starke Haftung des Gummis am metallischen Festigkeitsträger ermöglicht.

Durch die oben genannten X-Funktionalitäten wird durch Adsorption eine starke Bindung zur metallischen oder metalloxidischen Oberfläche erzielt. Als metallische Substrate können herkömmliche reine Metalle wie z. B. Aluminium, insbesondere auch die Elemente der Nebengruppen des Periodensystems (z. B. Platin, Titanium, Silber, Kupfer, Nickel, Zink, Eisen), Legierungen wie z. B. Stahl, Messing, als auch Metalloxide, wie z. B. Aluminiumoxid, Eisenoxid, verwendet werden. Durch die starke Adsorptionskraft der X-Funktionalität zur metallischen Oberfläche ist es möglich, eine monomolekulare Bedeckung an Haftvermittlersubstanz auf der metallischen Oberfläche aufzubringen. Demzufolge wird wenig Haftvermittlersubstanz benötigt, was kostengünstig ist. Als besonders vorteilhaft hat sich erwiesen, daß zur Haftung zum metallischen Substrat hin die Haftvermittlersubstanz eine Thiolgruppe (-SH) als X aufweist, die im günstigsten Fall eine kovalente Bindung zur Metalloberfläche ausbildet. Es wurde gefunden, daß besonders bei diesen Verbindungen eine weitgehend spontane Adsorption an der metallischen Oberfläche auftritt, die zu einer monomolekularen Bedeckung der metallischen Oberfläche führt.

Als weiterer Strukturbaustein der erfindungsgemäßen Verbindungsklasse dient R (Alkylrest, fluorierter Alkylrest, Alkylrest bzw. fluorierter Alkylrest mit oder ohne Säureamidgruppierung dazwischen) als "Spacer". Je nach der Größe von n und m kann die Kettenlänge von R variiert werden. Dadurch wird die mechanische Stabilität (Kettenbeweglichkeit) der Bindung beeinflußt, so daß eine optimale Anpassung an die Art der metallischen Oberfläche bzw. durch Variation von m an das vulkanisierbare Polymer erfolgen kann. Bevorzugt ist eine ―CH₂- oder ―CF₂-Gruppierung mit n, m ≤ 12, wodurch eine optimale Haftvermittlung erzielt wird.

Als aromatische und/oder heteroaromatische Systeme können aus der Literatur herkömmliche Verbindungen verwendet werden, insbesondere Oligophenyle und Oligothiophene, verwendet werden. Dabei können bis zu 6 aromatische Einheiten, bevorzugt bis zu 3, vorliegen. Bevorzugt befinden sich an diesem aromatischen und/oder heteroaromatischen System Substituenten, die deutlich elektronendrückend sind, wie z. B. die NH₂-Gruppierung oder die OCH₃-Gruppierung, oder aber auch elektronenziehende Substituenten, wie z. B. die CN-Gruppierung oder die NO₂-Gruppierung. Je nach Art des Substituenten können diese wiederum Einfluß auf die Bindung zur metallischen Oberfläche oder zum vulkanisierbaren Polymer aufweisen. Als Beispiele seien für Ar an dieser Stelle auch Benzen, Anilin oder Pyrrol genannt.

Als günstig hat sich ein aromatisches und/oder heteroaromatisches System (Ar) erwiesen, das ein Thiophen enthält. Der Thiophenbaustein der Haftvermittlersubstanz kann wie bereits allgemein für das aromatische und/oder heteroaromatische System erwähnt, ebenfalls mit geeigneten Substituenten versehen sein. Der Thiophenring kann dabei direkt an R gebunden sein. Es ist aber auch möglich, den Thiophenring mit oder ohne geeigneten Substituenten an ein bereits vorhandenes aromatisches System anzubinden. Vorteilhafterweise hat sich insbesondere für das Thiophen als "Anbindungsverfahren" an ein bereits vorhandenes aromatisches System das Verfahren der Elektrodeposition bewährt. Bei diesem elektrochemischen Prozeß wird angenommen, daß dem Thiophen unter dem Einfluß einer elektrischen Spannung Elektronen entzogen werden. Das entstandene Thiophenradikal ist in der Lage, sich unter Abspaltung eines Wasserstoffprotons mit dem bereits vorhandenen aromatischen System zu verbinden. Da die Spannung, die dazu benötigt wird, um dem Thiophen ein Elektron zu entreißen, relativ gering ist, ist das Thiophen für eine Anbindung an ein bereits vorhandenes aromatisches System durch Elektrodeposition ("Grafting") besonders geeignet. Prinzipiell können aber auch andere System, z. B. auch andere monomere oder oligomere Bausteine, mittels dieses Verfahrens gebunden werden.

Wie bereits erwähnt, weist die erfindungsgemäße Haftvermittlersubstanz eine Funktionalität Y auf, die es ermöglicht, mit dem vulkanisierbaren Polymer eine kovalente Bindung einzugehen. Y kann z. B. ein ungesättigter Rest wie z.B. Styren- oder Isoprengruppierung; -CH=CH₂ ; -CH=CHCl; -CH=CH-D, mit D verzweigter oder unverzweigter Alkylrest (z. B. Methyl-, Ethyl-, Propylrest) sein.

Je nach Art des zu vulkanisierenden Polymers kann die Funktionalität Y so gewählt werden, daß es während der Vulkanisation zu einer festen Bindung zwischen Polymer und Haftvermittlungssubstanz und somit auch zum Festigkeitsträger kommt. Als vulkanisierbare Polymere können sowohl Elastomere als auch thermoplastische Elastomere verwendet werden. Als Elastomere können z. B. Naturkautschuk, Styren-Butadien-Kautschuk, Butadienkautschuk, Butylkautschuk, Acrylnitril-Butadien-Kautschuk, Ethylen-Propylen-Dien-Copolymer oder Ethylen-Propylen-Copolymer zum Einsatz kommen. Als thermoplastisches Elastomer können aus dem Stand der Technik bekannte Polymere verwendet werden. Durch die Aufbringung des erfindungsgemäßen Haftvermittlers auf den metallischen Festigkeitsträger ist es möglich, eine hervorragende Bindung zumindest zwischen dem Elastomerblock des thermoplastischen Elastomers und dem Haftvermittler über dessen Funtionalität Y herzustellen. Es wird bereits so eine ausreichende Haftung zwischen dem thermoplastischen Elastomer und dem Festigkeitsträger erzielt.
Es ist möglich, die Vulkanisation durch Schwefel oder durch Peroxide zu aktivieren. Insbesondere bei der Schwefelvulkanisation muß die Funktionalität Y der Haftvermittlungssubstanz Doppel- oder Dreifachbindungen aufweisen, um mit dem Polymer eine Bindung eingehen zu können.

Für das Herstellen eines Verbundes aus metallischem Festigkeitsträger, Haftvermittlersubstanz und vulkanisierbarem Polymer ist es vorteilhaft, wenn die Haftvermittlersubstanz in einem organischen Lösungsmittel gelöst vorliegt, und der metallische Festigkeitsträger durch diese Lösung gezogen wird (Dip-Prozeß). Dabei kann die Haftvermittlersubstanz bereits alle Funktionalitäten (X, Y), die zur Haftung sowohl an den Festigkeitsträger als auch an das vulkanisierbare Polymer erforderlich sind, aufweisen. Die mit der Haftvermittlersubstanz beschichteten metallischen Festigkeitsträger können dann mit einem vulkanisierbaren Polymer beschichtet werden und anschließend der Vulkanisation unterzogen werden. Es ist aber auch möglich, der Dip-Lösung eine Haftvermittlersubstanz zuzugeben, die noch nicht die vollständige Struktur des erfindungsgemäßen Haftvermittlerstoffes aufweist, so daß der metallische Festigkeitsträger zuerst mit der ihm zugesprochenen Funktionalität (X) der Haftvermittlersubstanz zumindest bedeckt wird. Anschließend kann dann z. B. durch Elektrodendeposition der restliche Strukturteil der Haftvermittlersubstanz zumindest mit der Funktionalität (Y) an den bereits am metallischen Festigkeitsträger haftenden Teil der Haftvermittlersubstanz angebunden werden. Danach kann, wie bereits erwähnt, das vulkanisierbare Polymer aufgebracht und vulkanisiert werden.

Prinzipiell sind aber auch noch andere herkömmliche Aufbringungsmöglichkeiten des Haftvermittlers auf den metallischen Festigkeitsträger möglich, wie z. B. das Überführen der Haftvermittlersubstanz in eine Gasphase und anschließende Abscheidung der Substanz auf dem metallischen Festigkeitsträger.

Durch die Verwendung der erfindungsgemäßen Haftvermittlersubstanz, wobei in der Regel eine monomolekulare Bedeckung der Haftvermittlersubstanz auf dem metallischen Festigkeitsträger ausreicht, wird erzielt, daß nur wenig von dieser Substanz benötigt wird, um den Festigkeitsträger vollständig zu bedecken, so daß durch die Verwendung des erfindungsgemäßen Haftvermittlers auch eine kostengünstigere Herstellung eines metallverstärkten Gummiproduktes möglich ist. Durch die Variation der Bausteine R und Ar sowie n und m ist es möglich, auf die Funktionalitäten X und Y so einzuwirken, daß eine Variation der Bindungsstärken der Haftvermittlersubstanz zum metallischen Festigkeitsträger bzw. zum vulkanisierbaren Polymer gewährleistet ist. Demzufolge können verschiedenartige metallische Festigkeitsträger mit verschiedenen vulkanisierbaren Polymeren beschichtet werden, die eine hervorragende Haftung zwischeneinander aufweisen. Prinzipiell ist aber nicht einmal eine monomolekulare Bedeckung der Haftvermittlersubstanz auf dem metallischen Festigkeitsträger erforderlich (z.B. bei thermoplastischen Elastomeren). Durch die Funktionalitäten X und Y bewirken Adsorptionskräfte bzw. kovalente Bindungen eine feste Anbindung. Die Bindungsstärke einer kovalenten Bindung ist wesentlich größer als bei einer mechanischen Verzahnung, die bei herkömmlichen Messingbeschichtungen die Haftung bewirkt, so daß erfindungsgemäß eine festere Haftung erzielt wird. Außerdem wird der Nachteil der mechanischen Verzahnung, nämlich eine mangelnde Alterungsbeständigkeit des Verbundes durch den Einsatz der erfindungsgemäßen Haftvermittlersubstanz ausgeschaltet. Erfindungsgemäß können jetzt Gummiprodukte, die mit metallischen Festigkeitsträgern verstärkt sind, hergestellt werden, die korrosionsbeständiger sind und bessere mechanische Eigenschaften aufweisen. Bei mit Schwefel vulkanierbaren Polymeren ist es jetzt nicht mehr erforderlich, eine zusätzliche Zugabe von Schwefel in die Kautschukmischung einzubringen, der bisher für eine ausreichende Haftschichtausbildung für vermessingten Festigkeitsträger erforderlich war. Durch den Einsatz des erfindungsgemäßen Haftvermittlers kann sogar auf eine gesonderte Haftmischung verzichtet werden.

Der erfindungsgemäße Haftvermittler kann für sämtliche Gummiprodukte, die mit metallischen Festigkeitsträgern verstärkt sind, eingesetzt werden, so z. B. für Fördergurte, Luftfedern, Riemen, Schläuche und insbesondere für Fahrzeugreifen. Bei letzterem, bei dem Drahtkerne (im allgemeinen Vollgummireifen und Luftreifen) und/oder Gürtellagen und/oder Karkasse aus Stahl bestehen, wurden durch den Einsatz des Haftvermittlers besondere Vorteile erzielt. Durch das Weglassen der Haftmischungen können bei der Herstellung des Fahrzeugreifens die einzelnen Kautschukmischungen aufeinander optimal abgestimmt werden, so daß es jetzt auch erfindungsgemäß z. B. möglich wird, die Laufflächenmischung direkt auf den Gürtel aufzubringen. Somit kann der Reifen kostengünstiger hergestellt werden und gleichzeitig in seinen Laufeigenschaften optimiert werden. Des weiteren hat der Reifen eine höhere Lebensdauer.

Nachfolgend wird ein Ausführungsbeispiel anhand einer Zeichnung näher erläutert.

Es zeigt:
- Figur 1: schematischer Ablauf der Herstellung eines vulkanisierten Verbundes aus Stahlcord und einer Gummimischung, bei dem als Haftvermittler die erfindungsgemäße Substanz eingesetzt wird.
- Figur 2: einen radialen Querschnitt durch einen Fahrzeugluftreifen

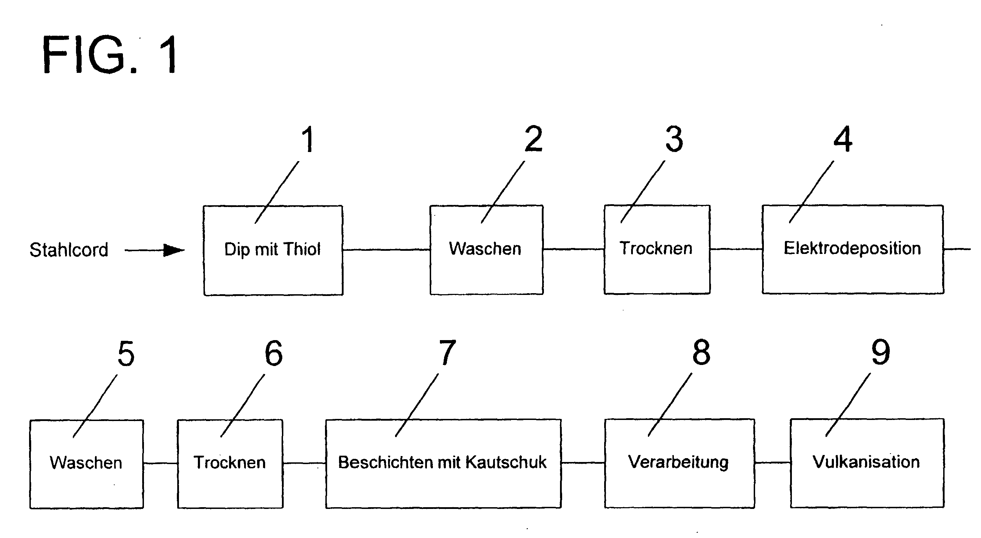

Im Schema der Figur 1 ist dargestellt, daß ein Cord eines beliebigen Stahltyps (z.B. 0,8 % Kohlenstoff, Spurenelemente) im Schritt 1 mit einer Lösung, im einfachsten Fall Thiophenol (Phenylmethanthiol, Phenylethanthiol, usw.) in Toluol, behandelt wird. Prinzipiell kann anstelle eines Thiols Alkylsiliziumchlorid, wie z. B. Trichlormethylsilan, oder Alkylsiloxane, wie z. B. Hexamethyldisiloxan oder organische Säure (oder organische Säurechloride verwendet werden. Die Konzentration des Thiols im Lösungsmittel Toluol ist z.B. 0,1 molar. Im Schritt 2 wird überflüssiges Thiol vom Stahlcord mit z.B. einer alkoholischen Lösung abgewaschen und im Schritt 3 einem Trocknungsprozeß bei ca. 60 °C unterzogen. Auf dem Stahlcord befindet sich nun ein Baustein der erfindungsgemäßen Haftvermittlungssubstanz, die durch eine kovalente Schwefelbindung auf dem Metall (Stahlcord) haftet. Durch Elektrodeposition (Schritt 4) wird nun der Teil der Haftvermittlungssubstanz (Y) an den bisher befindlichen Baustein der Haftvermittlungssubstanz am Festigkeitsträger gebunden. Dazu wird der behandelte Stahlcord in eine Dreielektrodenzelle, in der sich als 0,1 molare Badlösung das Thiophen (z.B. Vinylthiophen in Acetonitril) befindet, geführt, wobei der Stahlcord als eine von diesen gepolt wird. Eine weitere Elektrode wird als Referenz verwendet (Silber/Silbernitratelektrode) und als Arbeitselektrode ein Platinkristall. Die Spannung wird von 0 auf 0,75 V in 500 mV pro Sekunde-Schritten erhöht. Durch die angelegte Spannung wird dem Thiophen ein Elektron entrissen und das verbleibende Thiophenradikal ist in der Lage sich an den ersten Baustein der Haftvermittlersubstanz, die sich bereits auf dem Festigkeitsträger (Stahlcord) befindet unter Abstraktion eines Wasserstoffatoms anzulagern, wobei der Mechanismus der Anlagerung noch nicht geklärt ist.

Die Schritte 1 und 4 sind im folgenden noch einmal kurz zusammengefaßt:

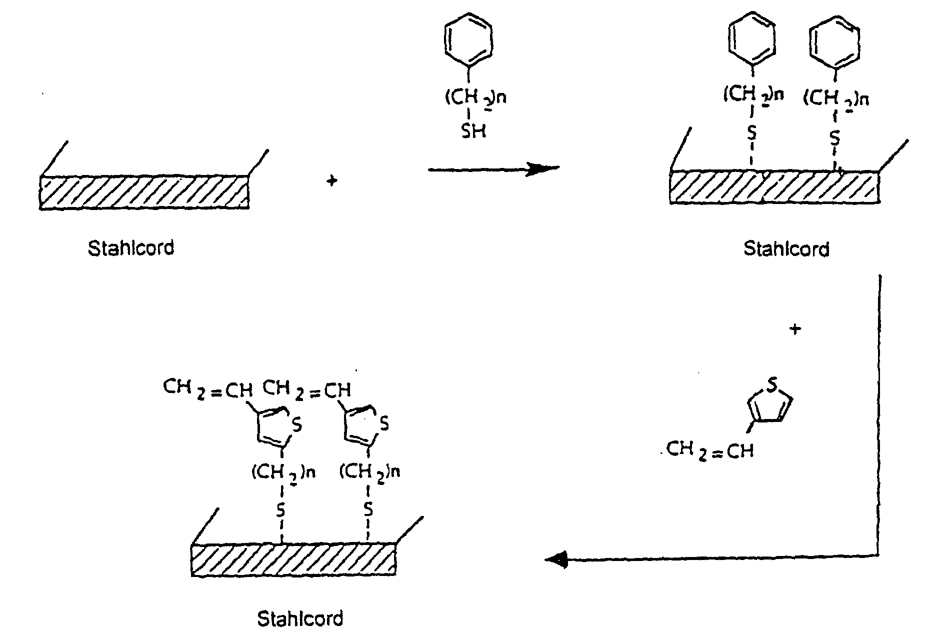

Nach der Elektrodeposition (Schritt 4) folgt ein weiterer Waschvorgang (Schritt 5) mit z.B. Wasser und ein Trocknungsprozeß (Schritt 6) bei ca. 60 °C. Die Schritte 4 bis 6 können auch weggelassen werden, wenn mit dem Dip-Vorgang (Schritt 1) bereits eine hinreichend reaktive Haftvermittlersubstanz auf den Stahlcord aufgebracht werden kann. Dazu muß die X- und Y-Funktionalität an der Haftvermittlersubstanz bereits vorhanden sein. Als Beispiel kommen folgende Verbindungen in Frage:

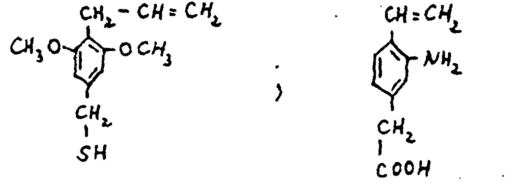

Nach dem Beschichten des Stahlcordes mit der Haftvermittlersubstanz folgt Schritt 7, nämlich das Aufbringen der unvulkanisierten Kautschukschicht. Dazu läuft in einem Kalander der mit Haftvermittler bedeckte Stahlcord ein und wird mit einer kalandrierten Kautschukplatte (z.B. Naturkautschukmischung) bedeckt. Dieser Verbund aus Stahlcord, Haftvermittlungssubstanz und Kautschukplatte kann in einem nächsten Verarbeitungsschritt 8 für die jeweilige Produktherstellung weiter verarbeitet werden. Für die Herstellung eines Fahrzeugluftreifens z. B. könnte der Verbund das Gürtelpaket darstellen, das während des Reifenbaus auf die Karkaßlage aufgelegt wird. Bei der weiteren Reifenherstellung wird auf das Gürtelpaket der unvulkanisierte Laufstreifen aufgebracht. Nach dem Fertigstellen des Reifenrohlings wird dieser in die Vulkanisierpresse gelegt und bei erhöhtem Druck und Temperatur vulkanisiert.

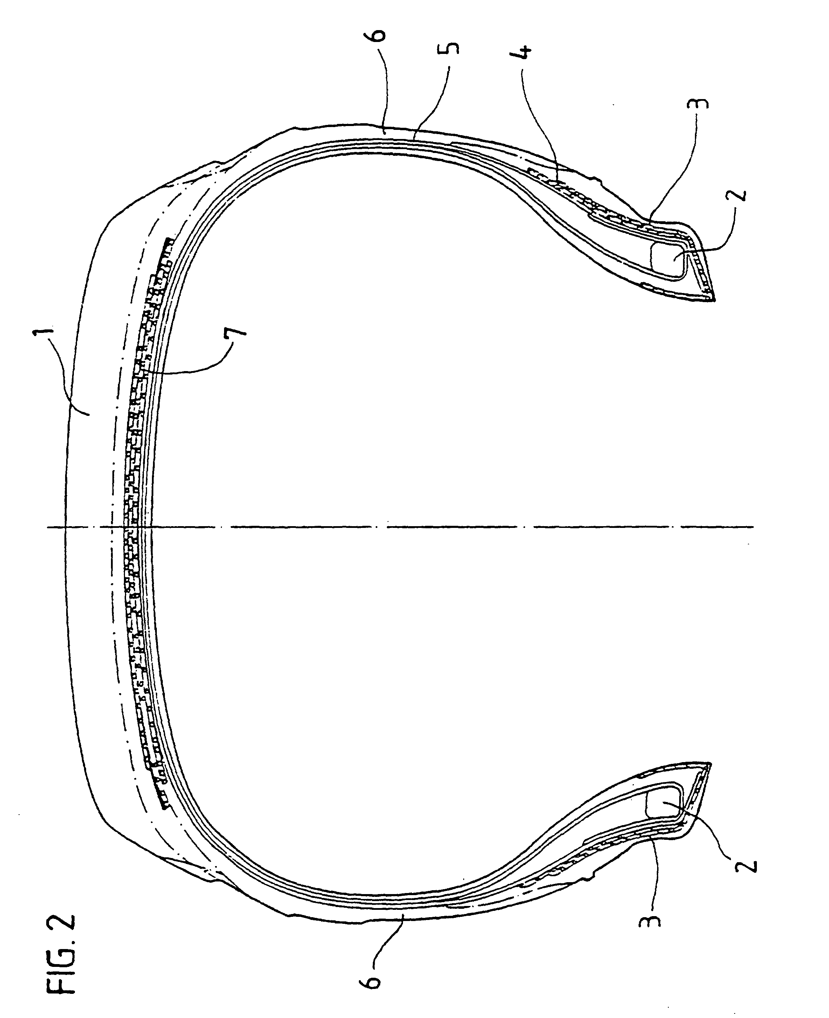

In der Figur 2 ist ein Fahrzeugluftreifen mit einem Laufstreifen 1, metallischem Wulstkern 2, der sich im Wulstbereich 3 befindet, einem Wulstverstärker 4, einer Karkasse 5, Seitenwänden 6 und einem metallischen Gürtel 7 dargestellt. Der Wulstkern 2 und der Gürtel 7 sind im allgemeinen aus metallischen Festigkeitsträgern. Aber auch die Karkasse 5 kann aus Stahlcord gefertigt sein. Insbesondere werden solche Reifen für den Einsatz bei Lastkraftwagen verwendet. Durch die Verwendung der erfindungsgemäßen Haftvermittlersubstanz können Reifen kostengünstiger hergestellt werden, da auf eine zusätzliche Haftmischung für metallische Festigkeitsträger verzichtet werden kann. Des weiteren können die einzelnen Kautschukmischungen besser aufeinander abgestimmt werden, so daß die Laufeigenschaften des Reifens optimiert werden können. Aufgrund der guten Haftung zwischen metallischem
Festigkeitsträger und Gummi werden Korrosionsprobleme weitgehend vermieden, so daß der Reifen höheren mechanischen Belastungen standhält und somit eine längere Lebensdauer aufweist.

## Patentansprüche

1. Haftvermittlersubstanz zur besseren Haftung zwischen einem vulkanisierbaren Polymer und einem metallischen Festigkeitsträger, **dadurch gekennzeichnet, daß** die Haftvermittlersubstanz folgende Struktur aufweist:
X - (R)ₙ - (Ar)ᵢ - (R)ₘ - Y,
wobei
X: -SH; -Si(Cl)₃; -Si[R₁(Cl)₂]; -Si(OR₁)₃; -Si[R₁(OR₁)₂]; -COOH; -COCl mit R₁ : Alkylrest (verzweigt oder unverzweigt) und
R: -CH₂-; -CF₂-; -CH₂-CO-NH-CH₂-; -CF₂-CO-NH-CF₂-; -CH₂-CO-NH-CF₂-; -CF₂-CO-NH-CH₂-
mit 0≤n,m ≤16 und
Ar: thiophenhaltiges System mit oder ohne Substituent
mit 0 < i ≤ 6, und
Y: ungesättigter Rest ist.

2. Haftvermittlersubstanz nach Anspruch 1, **dadurch gekennzeichnet, daß** X eine SH-Gruppe ist.

3. Haftvermittlersubstanz nach Anspruch 1 oder 2, **dadurch gekennzeichnet, daß** zumindest ein Teil des thiophenhaltigen Systems (Ar) mittels Elektrodeposition von monomeren oder oligomeren Bausteinen aufgebracht ist.

4. Haftvermittlersubstanz nach zumindest einem der vorhergehenden Ansprüche, **dadurch gekennzeichnet, daß** der Substituent am thiophenhaltigen System (Ar) eine Elektronendonor oder -akzeptorwirkung aufweist.

5. Verwendung einer Substanz mit der folgenden Struktur:
X - (R)ₙ - (Ar)ᵢ - (R)ₘ - Y,
wobei
X: -SH; -Si(Cl)₃; -Si[R₁(Cl)₂]; -Si(OR₁)₃; -Si[R₁(OR₁)₂]; -COOH; -COCl
mit R₁: Alkylrest (verzweigt oder unverzweigt) und
R: -CH₂-; -CF₂-; -CH₂-CO-NH-CH₂-; -CF₂-CO-NH-CF₂-; -CH₂-CO-NH-CF₂-; -CF₂-CO-NH-CH₂-
mit 0 ≤ n,m ≤ 16 und
Ar: aromatisches und/oder heteroaromatisches System mit oder ohne Substituent
mit 0 < i ≤6, und
Y: ungesättigter Rest ist,
als Haftvermittlersubstanz zur besseren Haftung zwischen einem vulkanisierbaren Polymer und einem metallischen Festigkeitsträger.

6. Verwendung nach Anspruch 5, **dadurch gekennzeichnet, daß** X bei der Substanz eine SH-Gruppe ist.

7. Verwendung nach Anspruch 5 oder 6, **dadurch gekennzeichnet, daß** das aromatisches und/oder heteroaromatisches System (Ar) bei der Substanz ein thiophenhaltiges System ist.

8. Verwendung nach zumindest einem der Ansprüche 5 bis 7, **dadurch gekennzeichnet, daß** der Substituent am aromatischen und/oder heteroaromatischen System (Ar) bei der Substanz eine Elektronendonor oder -akzeptorwirkung aufweist

9. Metallischer Festigkeitsträger zur Haftung an vulkanisierbarem Polymer, **dadurch gekennzeichnet, daß** der Festigkeitsträger mit einer Substanz der folgenden Struktur:
X - (R)ₙ - (Ar)ᵢ - (R)ₘ - Y,
wobei
X: -SH; -Si(Cl)₃; -Si[R₁(Cl)₂]; -Si(OR₁)₃; -Si[R₁(OR₁)₂]; -COOH; -COCl
mit R₁: Alkylrest (verzweigt oder unverzweigt) und
R: -CH₂-; -CF₂-; -CH₂-CO-NH-CH₂-; -CF₂-CO-NH-CF₂-; -CH₂-CO-NH-CF₂-; -CF₂-CO-NH-CH₂-
mit 0 ≤ n,m ≤ 16 und
Ar: aromatisches und/oder heteroaromatisches System mit oder ohne Substituent
mit 0<i≤6, und
Y: ungesättigter Rest ist,
als Haftvermittlersubstanz zur besseren Haftung zwischen dem vulkanisierbaren Polymer und dem Festigkeitsträger versehen ist.

10. Metallischer Festigkeitsträger zur Haftung an vulkanisierbarem Polymer nach Anspruch 9, **dadurch gekennzeichnet, daß** die Haftvermittlersubstanz auf den metallischen Festigkeitsträger mittels eines Dip-Vorganges aufgebracht ist.

11. Metallischer Festigkeitsträger zur Haftung an vulkanisierbarem Polymer nach Anspruch 9, **dadurch gekennzeichnet, daß** die Haftvermittlersubstanz auf den metallischen Festigkeitsträger durch eine Gasphasenreaktion aufgebracht ist.

12. Verbund aus vulkanisierbarem Polymer, einer Substanz der folgenden Struktur:
X - (R)ₙ - (Ar)ᵢ - (R)ₘ - Y,
wobei
X: -SH; -Si(Cl)₃; -Si[R₁(Cl)₂]; -Si(OR₁)₃; -Si[R₁(OR₁)₂]; -COOH; -COCI
mit R₁: Alkylrest (verzweigt oder unverzweigt) und
R: -CH₂-; -CF₂-; -CH₂-CO-NH-CH₂-; -CF₂-CO-NH-CF₂-; -CH₂-CO-NH-CF₂-; -CF₂-CO-NH-CH₂-
mit 0≤n,m≤ 16 und
Ar: aromatisches und/oder heteroaromatisches System mit oder ohne Substituent
mit 0<i≤6, und
Y: ungesättigter Rest ist,
als Haftvermittlersubstanz und metallischem Festigkeitsträger.

13. Verbund nach Anspruch 9, **dadurch gekennzeichnet, daß** das Polymer mit Schwefel vulkanisierbar ist.

14. Verbund nach Anspruch 12 oder 13, **dadurch gekennzeichnet, daß** der Verbund das Gürtelpaket eines Fahrzeugluftreifens darstellt.

## Claims

1. Adhesion promoter substance for better adhesion between a vulcanizable polymer and a metallic reinforcement, **characterized in that** the adhesion promoter substance has the following structure:
X - (R)ₙ - (AR)ᵢ - (R)ₘ - Y,
in which
X is -SH; -Si(Cl)₃; -Si[R₁(Cl)₂]; -Si(OR₁)₃; -Si[R₁(OR₁)₂]; -COOH; -COCl
where R₁ is an alkyl radical (branched or straight-chain) and
R is -CH₂- ; -CF₂-; -CH₂-CO-NH-CH₂-;
-CF₂-CO-NH-CF₂-; -CH₂-CO-NH-CF₂-; -CF₂-CO-NH-CH₂- where 0 ≤ n, m ≤ 16 and
Ar is a thiophene-containing system with or without a substituent
where 0 < i ≤ 6, and
Y is an unsaturated radical.

2. Adhesion promoter substance according to Claim 1, **characterized in that** X is an SH group.

3. Adhesion promoter substance according to Claim 1 or 2, **characterized in that** at least a part of the thiophene-containing system (Ar) is applied by means of electrodeposition of monomeric or oligomeric building blocks.

4. Adhesion promoter substance according to at least one of the preceding claims, **characterized in that** the substituent on the thiophene-containing system (Ar) has an electron donor or electron acceptor effect.

5. Use of a substance having the following structure:
X - (R)ₙ - (AR)ᵢ - (R)ₘ - Y,
in which
X is -SH; -Si(Cl)₃; -Si[R₁(Cl)₂]; -Si(OR₁)₃; -Si[R₁(OR₁)₂]; -COOH; -COCl
where R₁ is an alkyl radical (branched or straight-chain) and
R is -CH₂-; -CF₂-; -CH₂-CO-NH-CH₂-; -CF₂-CO-NH-CF₂-; -CH₂-CO-NH-CF₂-; -CF₂-CO-NH-CH₂-
where 0 ≤ n, m ≤ 16 and
Ar is an aromatic and/or heteroaromatic system with or without a substituent
where 0 < i ≤ 6, and
Y is an unsaturated radical,
as an adhesion promoter substance for improving adhesion between a vulcanizable polymer and a metallic reinforcement.

6. Use according to Claim 5, **characterized in that** X in the substance is an SH group.

7. Use according to Claim 5 or 6, **characterized in that** the aromatic and/or heteroaromatic system (Ar) in the substance is a thiophene-containing system.

8. Use according to at least one of Claims 5 to 7, **characterized in that** the substituent on the aromatic and/or heteroaromatic system (Ar) in the substance has an electron donor or electron acceptor effect.

9. Metallic reinforcement for adhesion to a vulcanizable polymer, **characterized in that** the reinforcement is provided with a substance of the following structure:
X - (R)ₙ - (Ar)ᵢ - (R)ₘ - Y,
in which
X is -SH; -Si(Cl)₃; -Si[R₁(Cl)₂]; -Si(OR₁)₃; -Si[R₂(OR₁)₂]; -COOH; -COCl
where R₁ is an alkyl radical (branched or straight-chain) and
R is -CH₂-; -CF₂-; -CH₂-CO-NH-CH₂-; -CF₂-CO-NH-CF₂-; -CH₂-CO-NH-CF₂-; -CF₂-CO-NH-CH₂-
where 0 ≤ n, m ≤ 16 and
Ar is an aromatic and/or heteroaromatic system with or without a substituent
where 0 < i ≤ 6, and
Y is an unsaturated radical,
as an adhesion promoter substance for improving adhesion between the vulcanizable polymer and the reinforcement.

10. Metallic reinforcement for adhesion to a vulcanizable polymer according to Claim 9, **characterized in that** the adhesion promoter substance is applied to the metallic reinforcement by means of a dip process.

11. Metallic reinforcement for adhesion to a vulcanizable polymer according to Claim 9, **characterized in that** the adhesion promoter substance is applied to the metallic reinforcement by a gas-phase reaction.

12. Composite of vulcanizable polymer, a substance of the following structure:
X - (R)ₙ - (Ar)ᵢ - (R)ₘ - Y,
in which
X is -SH; -Si(Cl)₃; -Si(R₁(Cl)₂]; -Si(OR₁)₃; -Si[R₁(OR₁)₂]; -COOH; -COCl
where R₁ is an alkyl radical (branched or straight-chain) and
R is -CH₂-; -CF₂-; -CH₂-CO-NH-CH₂-; -CF₂-CO-NH-CF₂-; -CH₂-CO-NH-CF₂-; -CF₂-CO-NH-CH₂-
where 0 ≤ n, m ≤ 16 and
Ar is an aromatic and/or heteroaromatic system with or without a substituent
where 0 < i ≤ 6, and
Y is an unsaturated radical,
as an adhesion promoter substance, and a metallic reinforcement.

13. Composite according to Claim 9, **characterized in that** the polymer is vulcanizable with sulphur.

14. Composite according to Claim 12 or 13, **characterized in that** the composite represents the belt package of a pneumatic aircraft tyre.

## Revendications

1. Substance promotrice d'adhérence pour la meilleure adhérence entre un polymère vulcanisable et un renfort métallique, **caractérisée en ce que** la substance promotrice d'adhérence présente la structure suivante :
X-(R)ₙ(Ar)ᵢ-(R)ₘ-Y,
où
X : -SH ; -Si(Cl)₃ ; -Si[R₁(Cl)₂] ; -Si(OR₁)₃ ; -Si[R₁(OR₁)₂], -COOH ; -COCl,
R₁ représentant un radical alkyle (ramifié ou non ramifié) et
R : -CH₂- ; -CF₂- ; -CH₂-CO-NH-CH₂- ; -CF₂-CO-NH-CF₂- ; -CH₂-CO-NH-CF₂- ; -CF₂-CO-NH-CH₂-
avec 0≤n, m≤16 et
Ar représente un système contenant un groupe thiophène avec ou sans substituant,
avec 0 < i ≤ 6, et
Y représente un radical insaturé.

2. Substance promotrice d'adhérence selon la revendication 1, **caractérisée en ce que** X est un groupe SH.

3. Substance promotrice d'adhérence selon la revendication 1 ou 2, **caractérisée en ce qu'**au moins une partie du système (Ar) contenant un groupe thiophène est appliquée par électrodéposition de composants monomères ou oligomères.

4. Substance promotrice d'adhérence selon au moins une des revendications précédentes, **caractérisée en ce que** le substituant sur le système (Ar) contenant un groupe thiophène présente une activité de donneur ou d'accepteur d'électrons.

5. Utilisation d'une substance ayant la structure suivante :
X-(R)ₙ(Ar)ᵢ-(R)ₘ-Y,
où
X : -SH ; -Si(Cl)₃; -Si[R₁(Cl)₂]; -Si(OR₁)₃; -Si[R₁(OR₁)₂], -COOH ; -COCl,
R₁ représentant un radical alkyle (ramifié ou non ramifié) et
R : -CH₂- ; -CF₂- ; -CH₂-CO-NH-CH₂- ; -CF₂-CO-NH-CF₂-; -CH₂-CO-NH-CF₂- ; -CF₂-CO-NH-CH₂-
avec 0≤n,m≤16 et
Ar : représente un système aromatique et/ou hétéroaromatique avec ou sans substituant,
avec 0<i≤6, et
Y : représente un radical insaturé,
en tant que substance promotrice d'adhérence pour la meilleure adhérence entre un polymère vulcanisable et un renfort métallique.

6. Utilisation selon la revendication 5, **caractérisée en ce que** X dans la substance est un groupe SH.

7. Utilisation selon la revendication 5 ou 6, **caractérisée en ce que** le système aromatique et/ou hétéroaromatique (Ar) dans la substance est un système contenant un groupe thiophène.

8. Utilisation selon au moins une des revendications 5 à 7, **caractérisée en ce que** le substituant sur le système aromatique et/ou hétéroaromatique (Ar) dans la substance présente une activité de donneur ou d'accepteur d'électrons.

9. Renfort métallique destiné à adhérer à un polymère vulcanisable, **caractérisé en ce que** le renfort est muni d'une substance ayant la structure suivante :
X-(R)ₙ-(Ar)ᵢ-(R)ₘY,
où
X : -SH ; -Si(Cl)₃ ; -Si[R₁(Cl)₂]; -Si(OR₁)₃; -Si[R₁(OR₁)₂], -COOH ; -COCl,
R₁ représentant un radical alkyle (ramifié ou non ramifié) et
R : -CH₂- ; -CF₂- ; -CH₂-CO-NH-CH₂-; -CF₂-CO-NH-CF₂- ; -CH₂-CO-NH-CF₂-; -CF₂-CO-NH-CH₂-
avec 0≤n,m≤16 et
Ar : représente un système aromatique et/ou hétéroaromatique avec ou sans substituant,
avec 0<i≤6, et
Y : représente un radical insaturé,
en tant que substance promotrice d'adhérence pour la meilleure adhérence entre le polymère vulcanisable et le renfort métallique.

10. Renfort métallique destiné à adhérer à un polymère vulcanisable selon la revendication 9, **caractérisé en ce que** la substance promotrice d'adhérence est appliquée sur le renfort métallique au moyen d'un processus de trempage.

11. Renfort métallique destiné à adhérer à un polymère vulcanisable selon la revendication 9, **caractérisé en ce que** la substance promotrice d'adhérence est appliquée sur le renfort métallique par une réaction en phase gazeuse.

12. Composite à base d'un polymère vulcanisable, d'une substance ayant la structure suivante :
X-(R)ₙ-(Ar)ᵢ-(R)ₘ-Y,
où
X : -SH ; -Si(Cl)₃; -Si[R₁(Cl)₂]; -Si(OR₁)₃; -Si[R₁(OR₁)₂], -COOH ; -COCl,
R₁ représentant un radical alkyle (ramifié ou non ramifié) et
R : -CH₂- ; -CF₂- ; -CH₂-CO-NH-CH₂- ; -CF₂-CO-NH-CF₂- ; -CH₂-CO-NH-CF₂- ; -CF₂-CO-NH-CH₂-
avec 0 ≤ n,m ≤ 16 et
Ar :représente un système contenant un groupe thiophène avec ou sans substituant,
avec 0<i≤6, et
Y : représente un radical insaturé,
en tant que substance promotrice d'adhérence, et d'un renfort métallique.

13. Composite selon la revendication 9, **caractérisé en ce que** le polymère est vulcanisable au soufre.

14. Composite selon la revendication 12 ou 13, **caractérisé en ce que** le composite représente la carcasse d'un pneumatique de véhicule.
